(19) Europäisches Patentamt

European Patent Office

Office européen des brevets

(11) **EP 1 433 482 B1**

(12) **EUROPEAN PATENT SPECIFICATION**

(45) Date of publication and mention
of the grant of the patent:
**20.12.2006 Bulletin 2006/51**

(51) Int Cl.:
*A61K 31/737* (2006.01)     *A61K 33/24* (2006.01)
*A61P 43/00* (2006.01)     *A61K 47/12* (2006.01)
*A61K 9/00* (2006.01)

(21) Application number: **03029710.5**

(22) Date of filing: **23.12.2003**

(54) **Aqueous preparation containing a shark-derived chondroitin iron sulfate colloid**

Wässerige Zubereitung von einem Kolloid aus Eisen-Haichondroitinsulfat

Préparation aqueuse de colloïde de chondroitine sulfate de fer, extrait de requin

(84) Designated Contracting States:
**AT BE BG CH CY CZ DE DK EE ES FI FR GB GR HU IE IT LI LU MC NL PT RO SE SI SK TR**

(30) Priority: **27.12.2002 JP 2002381167**

(43) Date of publication of application:
**30.06.2004 Bulletin 2004/27**

(73) Proprietor: **NIPRO CORPORATION**
Kita-ku, Osaka 531-8510 (JP)

(72) Inventors:
• **Nishida, Seiji,**
**Nipro Corporation**
**Osaka-shi**
**Osaka 531-8510 (JP)**
• **Katayama, Naohisa,**
**Nipro Corporation**
**Osaka-shi**
**Osaka 531-8510 (JP)**
• **Katsumata, Takashi,**
**Nipro Corporation**
**Osaka-shi**
**Osaka 531-8510 (JP)**
• **Sato, Makoto,**
**Nipro Corporation**
**Osaka-shi**
**Osaka 531-8510 (JP)**

(74) Representative: **Albrecht, Thomas et al**
**Kraus & Weisert,**
**Thomas-Wimmer-Ring 15**
**80539 München (DE)**

(56) References cited:
**EP-A- 1 308 155**     **WO-A-93/09766**

• **RONCA G ET AL: "Metabolic fate of partially depolymerized shark chondroitin sulfate in man"** INTERNATIONAL JOURNAL OF CLINICAL PHARMACOLOGY RESEARCH, BIOSCIENCE EDIPRINT, GENEVA,, CH, vol. XIII, no. Suppl, 1993, pages 27-34, XP009006912 ISSN: 0251-1649
• **DATABASE WPI Section Ch, Week 198333** Derwent Publications Ltd., London, GB; Class B04, AN 1983-738544 XP002272582 & JP 58 116413 A (NIPPON KAYAKU KK), 11 July 1983 (1983-07-11)
• **PATENT ABSTRACTS OF JAPAN vol. 2000, no. 09, 13 October 2000 (2000-10-13) & JP 2000 178181 A (NISSHO CORP), 27 June 2000 (2000-06-27)**
• **NARAZAKI M.; EGUCHI K.; SEKIBA K.: '"Chorionic transfer of iron, an experimental study in the human placenta"'** ACTA NEONATOLOGICA JAPONICA vol. 17, no. 3, 1981, pages 451 - 456, XP008066356

Remarks:
The file contains technical information submitted after the application was filed and not included in this specification

**Description**

Technical Field of the Invention

The present invention relates to an aqueous preparation containing essential trace elements for high-calorie intravenous nutrition infusion which is capable of preventing and treating various deficiency symptoms derived from a lack of essential trace elements. More specifically, the present invention relates to an aqueous preparation for high-calorie intravenous nutrition infusion having excellent pharmaceutical safety and pharmaceutical stability containing essential trace elements such as iron, selenium, zinc, copper, iodine, and/or manganese, used for infusion therapy.

Background of the Invention

Conventionally, high-calorie intravenous nutrition infusion therapy is typically performed and is widely used for a patient who has no or insufficient oral or enteral feeding ability. In high-calorie intravenous nutrition infusion therapy, generally, carbohydrates or the like as a source of energy, amino acids as a source of protein, and other components such as electrolytes and vitamins are intravenously administered. However, when a high-calorie intravenous nutrition infusion containing the above components is used for a long period of time, deficiency symptoms accompanied by a lack of essential trace elements for humans, namely, iron, selenium, zinc, copper, iodine, manganese, molybdenum, and chromium, develop.

When iron is parenterally supplied, there is a problem in terms of toxicity because an ionic iron compound binds to transferrin under a saturation condition and also binds to a plasma protein, causing shock or the like. Thus, there is a need to devise a means of supplying iron in a colloidal form with few side effects. As iron ion to be parenterally supplied to humans, ferric chloride is generally used. In an aqueous solution, such ferric chloride exists as a ferric hydroxide colloid particle. Such a colloid particle includes oxy chloride (FeOCl) in addition to ferric oxide ($Fe_2O_3$) and water; is a hydrophobic colloid positively charged by dissociation to $FeO^+$ and $Cl^-$; and has a tendency to aggregate. If the pH value thereof rises to about 3 or more, it will precipitate as a result of such aggregation (See, for example, "Colloid Chemistry", written by B. Jirgensons et al., and translated under the editorship of Bunichi Tamamushi, Baifukan, Tokyo, 1967).

In Japan, as a protective colloid, an iron colloid solution containing a chondroitin sulfate having few side effects and high iron utilization ratio has been used. For example, chondroitin iron sulfate colloid is commercially available as an intravenous injection preparation for iron deficiency anemia under the name Blutal (trade name, Dainippon Pharmaceutical Co., Ltd.). In addition, a preparation containing chondroitin iron sulfate colloid as a supplement of essential trace elements for high-calorie intravenous nutrition is also commercially available as Elemenmic injection and Elemate injection (trade names, Ajinomoto Pharma, Co., Ltd.), Mineralin injection and Parmirin injection (trade names, Nippon Pharmaceutical Co., Ltd./Takeda Chemical Industries, Ltd.), and the like.

Each of these commercially available preparations containing chondroitin iron sulfate colloid is prepared using a bovine-derived sodium chondroitin sulfate as a protective colloid. In 1996, however, it was announced in Great Britain that there was a causal relationship between the onset in a patient of Variant Creutzfeldt-Jakob disease (vCJD) and mad cow disease, i.e., bovine spongiform encephalopathy (BSE). An abnormal prion protein regarded as a cause of BSE is heat-stable. Therefore, it is considered that a high-temperature, high-pressure treatment and an alkali treatment will not perfectly inactivate the prion protein.

In Japan, in consideration of the outbreak of bovine BSE in Europe, for any drug or the like manufactured using a raw material derived from cows or the like, there is a need that manufacturers and so on take measures for ensuring quality and safety. Accordingly, a medicine security chief of the Ministry of Health, Labour, and Welfare published a bureau notice No. 1226, "For ensuring qualities and safeties of drugs and so on manufactured using a bovine-derived product or the like as a raw material" dated December 12, 2000, requiring guided self inspection, preparation of written acknowledgement, and so on by drug manufacturers or the like.

Bovine-derived chondroitin sulfate has been isolated and purified from bovine tracheae. However, there is a demand to use a safer chondroitin sulfate replacing a bovine-derived product.

An object of the present invention is to provide an aqueous preparation according to claim 1 containing a shark-derived chondroitin iron sulfate colloid not containing any BSE causative agent, which is safe and excellent in pharmaceutical stability, and additionally is stable when mixed in an infusion such as a high-calorie intravenous nutrition infusion,

more specifically, in the present invention, an aqueous preparation according to claim 1 containing a shark-derived chondroitin iron sulfate colloid prepared using, instead of a bovine-derived sodium chondroitin sulfate, a shark-derived sodium chondroitin sulfate not containing any BSE causative agent and which is safe, inhibits the decline of a pH value after sterilization and improves heat stability.

Summary of the Invention

The inventors of the present invention have carefully considered various ways of obtaining an aqueous preparation not containing any BSE causative agent, which is safe and excellent in pharmaceutical stability, and additionally is stable when mixed in an infusion such as a high-calorie intravenous nutrition infusion. As a result, the inventors of the present invention have found that an aqueous preparation containing a shark-derived chondroitin iron sulfate colloid in which shark-derived sodium chondroitin sulfate is provided as a protective colloid and a pH buffer inhibits the decline of pH after sterilization and is excellent in stability. By devoting themselves to intensive research relating to these findings, the present invention has finally been completed.

That is, the present invention relates to:

(1) a stabilized aqueous preparation comprising a shark-derived chondroitin iron sulfate colloid and a trace element, further comprising a pH buffer, wherein the shark-derived chondroitin iron sulfate colloid is produced from a shark-derived chondroitin sulfate : wherein the shark-derived sodium chondroitin sulfate has an average molecular weight of from 10,000 to 25,000; a limiting viscosity of from 0.27 to 0.65 dl/g; and a sulfur content of from 6.4 to 7.0 wt/wt%, wherein the pH buffer is one or more compounds selected from the group consisting of acetic acid, malonic acid, maleic acid, malic acid, tartaric acid, citric acid, L-sodium glutamate, disodium phthalate, ,

(2) an aqueous preparation as described in item (1) above, wherein the shark-derived chondroitin sulfate is an alkali metal salt of the shark-derived chondroitin sulfate,

(3) an aqueous preparation as described in item (1) above, wherein the shark-derived chondroitin sulfate is shark-derived sodium chondroitin sulfate,

(4) an aqueous preparation as described in item (1) above, wherein the pH buffer is malic acid, citric acid or L-sodium glutamate,

(5) an aqueous preparation as described in item (1) above, wherein the trace element is one or more elements selected from the group consisting of selenium, zinc, copper, iodine, and manganese,

(6) an aqueous preparation as described in item (1) above, wherein the trace elements are zinc, copper, and iodine,

(7) an aqueous preparation as described in item (1) above, wherein the trace elements are zinc, copper, iodine, and manganese,

(8) an aqueous preparation as described in item (1) above, wherein the trace elements are selenium, zinc, copper, iodine, and manganese,

(9) an aqueous preparation as described in item (1) above, wherein a weight ratio of iron : shark-derived sodium chondroitin iron sulfate colloid is from 1:4 to 1:6,

(10) an aqueous preparation as described in item (9) above, wherein the weight ratio of iron : shark-derived chondroitin iron sulfate colloid is about 1:5,

(11) an aqueous preparation as described in item (1) above, wherein the concentration of the pH buffer is from 0.01 to 1.0 wt/vol%,

(12) an aqueous preparation as described in item (1) above, wherein the preparation contains a shark-derived chondroitin iron sulfate colloid produced by adding an aqueous ferric salt solution and an aqueous alkali metal hydroxide solution to an aqueous shark-derived chondroitin sulfate solution so that the pH of the resulting mixture is adjusted to a pH within a range of from 5.5 to 7.5,

(13) a method for manufacturing an aqueous preparation containing a shark-derived chondroitin iron sulfate colloid, comprising: adding an aqueous ferric salt solution and an aqueous alkali metal hydroxide solution to an aqueous shark-derived chondroitin sulfate solution so that the pH of the resulting mixture is adjusted to a pH within a range of from 5.5 to 7.5; heating the resulting mixture; and adding a pH buffer and a trace element; and

(14) a method for manufacturing an aqueous preparation containing a shark-derived chondroitin iron sulfate colloid as described in item (13) above, wherein the shark-derived chondroitin sulfate is shark-derived sodium chondroitin sulfate, and the alkali metal hydroxide is sodium hydroxide.

Detailed Description of the Invention

An aqueous preparation of the present invention, which includes a shark-derived chondroitin iron sulfate colloid, a trace element, and a pH buffer (hereinafter sometimes referred to as the aqueous preparation of the present invention), can be manufactured by adding an aqueous ferric salt solution and an aqueous alkali metal hydroxide solution to an aqueous shark-derived chondroitin sulfate solution so that the pH of the resulting mixture is adjusted to a pH within the range of from 5.5 to 7.5; heating the resulting mixture; and adding a pH buffer and a trace element.

The shark-derived chondroitin sulfate is, for example, an alkali metal salt such as a sodium salt or potassium salt of a shark-derived chondroitin sulfate and, preferably, is shark-derived sodium chondroitin sulfate. The shark-derived sodium chondroitin sulfate is one which is derived from shark cartilage and has such physical properties that an average molecular weight is from 10,000 to 25,000; a limiting viscosity is from 0.27 to 0.65 dl/g (measured by capillary tube viscometer); and a sulfur content is from 6.4 to 7.0 wt/wt%. Preferably, the shark-derived sodium chondroitin sulfate is one in which a composition ratio of chondroitin-4-sulfate (chondroitin sulfate A): chondroitin-6-sulfate (chondroitin sulfate C) is 1:3.

The chondroitin sulfate is a linear polymeric polysaccharide having a repetitive structure with disaccharide units of [→ 4-glucuronic acid β1 → 3N-acetyl-D-galactosamine β1 →] and is a poly anion having a high negative charge in which isomers are present depending on the number of sulfate groups bound to such disaccharide units and the binding positions thereof. Table 1 shows a comparison of each isomer-composition ratio of sodium chondroitin sulfate derived from shark cartilage and from bovine tracheae (an average molecular weight of 20,000 to 25,000).

Table 1

| Position of sulfate group | Isomer composition ratio | |
|---|---|---|
| | Shark-cartilage-derived Chs Molecular weight 20,000-25,000 | Bovine-trachea-derived Chs Molecular weight 15,000-20,000 |
| $\Delta$Di-0S | 4.4% | 5.1% |
| $\Delta$Di-4S | 21.0% | 47.5% |
| $\Delta$Di-6S | 60.4% | 43.0% |
| $\Delta$Di-diS$_D$ | 12.1% | 1.1% |
| $\Delta$Di-diS$_E$ | 2.0% | 0.7% |
| (Note) Chs: sodium chondroitin sulfate.<br>$\Delta$Di-0S: A sulfate group is not bonded.<br>$\Delta$Di-4S: A sulfate group is bonded to the C-4 position of N-acetyl-D-galactosamine.<br>$\Delta$Di-6S: A sulfate group is bonded to the C-6 position of N-acetyl-D-galactosamine.<br>$\Delta$Di-diS$_D$: A sulfate group is bonded to the C-6 position of N-acetyl-D-galactosamine, and a sulfate group is bonded to the C-2 position of D-glucuronic acid.<br>$\Delta$Di-diS$_E$: Sulfate groups are bonded to the C-4 and C-6 positions of N-acetyl-D-galactosamine. | | |

Examples of the ferric salt include a compound containing ferric iron which can be used in the body such as ferric chloride hexahydrate ($FeCl_3.6H_2O$), ferric citrate ($FeC_6H_5O_7$), iron oxyhydroxide (FeO(OH)), ferric nitrate ($Fe(NO_3)_3.9H_2O$), iron oxide ($Fe_2O_3$), iron sulfate ($Fe_2(SO_4)_3.nH_2O$), or iron phosphate ($FePO_4.nH_2O$). Ferric salt changes to ferric hydroxide in an aqueous solution, and the shark-derived chondroitin sulfate is used as a protective colloid of the hydrophobic colloid solution of the ferric hydroxide. Of these, a ferric chloride such as ferric chloride hexahydrate ($FeCl_3.6H_2O$) is preferable. The weight ratio of iron:shark-derived sodium chondroitin sulfate is in the range of 1:4 to 1:6, and is preferably 1:5.

Examples of the alkali metal hydroxide include sodium hydroxide or potassium hydroxide, preferably sodium hydroxide.

The pH buffer to be used, having a function of buffering the pH value, is acetic acid, malonic acid, maleic acid, malic, acid, tartaric acid, citric acid, L-sodium glutamate, disodium phthalate. One or two or more of these compounds may be selected and used.

Examples of the trace elements include selenium, zinc, copper, iodine, and manganese. These metal elements usually exist in the preparation in ionic form. Preferably, the trace element comprises 3 elements of zinc, copper and iodine, 4 elements of zinc, copper, manganese and iodine, or 5 elements of zinc, copper, manganese, selenium and iodine.

Selenium used as the trace element includes compounds such as selenious acid, sodium selenite, and sodium selenate. Zinc salts such as sulfates and chlorides are used, and examples thereof include zinc sulfate heptahydrate and zinc chloride. Copper salts such as sulfates and chlorides are used, and examples thereof include copper sulfate pentahydrate, cuprous chloride, and cupric chloride. Iodine to be used includes compounds such as potassium iodide and sodium iodide. Manganese salts such as sulfates and chlorides are used, and examples thereof include manganese sulfate pentahydrate and manganese chloride tetrahydrate.

More specifically, the aqueous preparation of the present invention can be manufactured by adding an aqueous ferric salt solution and an aqueous alkali metal hydroxide solution to an aqueous shark-derived chondroitin sulfate solution so that the pH of the resulting mixture is adjusted to a pH within the range of from 5.5 to 7.5 and chondroitin iron sulfate colloid is formed; heating the mixture containing the chondroitin iron sulfate colloid; adding a pH buffer to the mixture after heating; adding one or two or more of a selenium compound, zinc salt, copper salt, iodide, and manganese salt; adjusting the pH to 5.8; and sterilizing the resultant under high-pressure steam. The temperature of heating is

4

preferably from about 100°C to 121°C. The time of heating is preferably from 10 to 30 minutes.

In the manufacturing process of the present invention, an aqueous preparation according to claim 1 containing a shark-derived chondroitin iron sulfate colloid and trace elements obtained by adding a base such as alkali metal hydroxide (preferably sodium hydroxide) as it is or as an aqueous solution without an addition of a pH buffer to adjust the pH of the final aqueous preparation to about 5.8 after the addition of trace element compounds, can be used in the same way as the other preparation of the present invention.

In the process of adding the trace elements, it is preferred that the solution is controlled so that the pH is from 5.3 to 6.3, preferably from 5.6 to 6.0, more preferably 5.8, after one or more compounds selected from the group consisting of a selenium compound, zinc salt, copper salt, iodide and manganese salt are added to the aqueous solution comprising a shark-derived chondroitin iron sulfate colloid. Then iodide is added to the solution.

In the manufacturing method of the present invention, an appropriate amount of the solution prepared by dissolving the ferric salt (e.g., ferric chloride hexahydrate) in a water for injection and an appropriate amount of the aqueous solution of alkali metal hydroxide (e.g., sodium hydroxide) are added with stirring to the solution prepared by dissolving shark-derived chondroitin sulfate (e.g., sodium chondroitin sulfate) in a water for injection, the amount of the shark-derived chondroitin sulfate corresponding to the above weight ratio of shark-derived sodium chondroitin sulfate to iron. It is preferable to adjust the pH of the mixture to any constant pH within the range of from 5.5 to 7.5.

The concentration of the aqueous ferric salt (e.g., ferric chloride hexahydrate) solution to be added is generally in the range of from 3 to 62 wt/vol%, preferably 13 to 32 wt/vol%.

The concentration of the aqueous alkali metal hydroxide (e.g., sodium hydroxide) solution to be added is generally in the range of from 1 to 28 wt/vol%, preferably from 2 to 7 wt/vol%.

The concentration of the aqueous chondroitin sulfate solution is generally in the range of from 3 to 30 wt/vol%, preferably 4 to 20 wt/vol%.

The concentration of the pH buffer to be used is generally 1 wt/vol% or less, preferably from 0.01 to 1 wt/vol%.

The mixture containing the ferric salt, the alkali metal hydroxide, and the chondroitin sulfate is stirred sufficiently to maintain the pH of the mixture to a pH within the range of 5.5 to 7.5 and to form the chondroitin iron sulfate colloid.

The time of reaction to form the chondroitin iron sulfate colloid may be appropriately determined by a person skilled in the art. In general, it may be in the range of from about an hour to about 6 hours. The temperature of the reaction may be appropriately selected by a person skilled in the art. Preferably, it is in the range of about 5°C to about 25°C.

The thus-obtained aqueous preparation of the present invention may be used as an injection after sterilization, if required. In addition, separate containers can be filled with the solution in small portions (e.g., 1, 2, or 4 mL each), sealed, and subjected to sterilization (e.g., high-pressure steam sterilization). Desirably, the aqueous preparation of the present invention has a pH in the range of from 4.5 to 6.0.

As the container to encapsulate the aqueous preparation of the present invention, for example, a glass container (such as an ampoule), and a container made of a plastic material such as polypropylene, including a pre-filled type syringe, may be used.

The injection of the present invention can be administered to humans safely, while scarcely causing any side effects, in accordance with methods known per se. The amount of elements contained in the aqueous preparation of the present invention to be administered is, as a daily dose per adult person, from about 0.9 to about 720 $\mu$mol of iron, from about 0.025 to about 5.0 $\mu$mol of selenium, from about 3.85 to about 210 $\mu$mol of zinc, from about 0.9 to about 55 $\mu$mol of copper, from 0 to about 11 $\mu$mol of iodine, and from 0 to about 51 $\mu$mol of manganese; preferably from about 9 to about 720 $\mu$mol of iron, from about 0.25 to about 2.5 $\mu$mol of selenium, from about 38.5 to about 61.5 $\mu$mol of zinc, from about 9.1 to about 27.3 $\mu$mol of copper, from about 0.6 to about 1.1 $\mu$mol of iodine, and from 0 to about 14.5 $\mu$mol of manganese. It is desirable that these elements are contained in from 2 to 20 mL of the aqueous solution. The aqueous preparation of the present invention may optionally include an additional element such as chromium, molybdenum, cobalt, and fluorine.

Examples

Hereinafter, the present invention will be described more specifically with reference to examples.

Example 1

An aqueous ferric chloride solution obtained by dissolving 94.6 g of ferric chloride into 390 mL of purified water were fed into a solution of shark chondroitin sulfate obtained by dissolving 97.74 g of shark-cartilage-derived sodium chondroitin sulfate (molecular weight of about 10,000) into 1480 mL of purified water, in a 5-fold amount of the shark-cartilage-derived sodium chondroitin sulfate to the amount of iron (Fe) in weight ratio, with stirring, while adding an amount of an aqueous sodium hydroxide solution necessary to maintain the pH at about 6.5, followed by purified water to adjust to a volume of 5000 mL. Subsequently, the resultant mixture was heated at 110□C for 20 minutes to prepare chondroitin iron sulfate colloid solution with 4 mg/mL of iron concentration. These operations were repeated once more and the total volume of the obtained chondroitin iron sulfate colloid solution was 10 L.

172.50 g of zinc sulfate were dissolved in 500 mL of purified water to prepare a zinc sulfate solution. Also, 12.48 g of copper sulfate and 1.66 g of potassium iodide were each dissolved in 250 mL of purified water to prepare a copper sulfate solution and a potassium iodide solution.

To 100 mL portions of the chondroitin iron sulfate colloid solution, an appropriate amount of purified water was added, and a pH buffer corresponding to respective concentrations as described below was added. If necessary, after adjusting the pH of each mixture to about 5.8, 10 mL of the zinc sulfate solution and 5 mL of the copper sulfate solution were added in order, and the pH was adjusted to 5.8 by adding an aqueous sodium hydroxide solution (1 wt/vol%). Further, 5 mL of the potassium iodide solution was added, and purified water was added to obtain a solutions that are each 400 mL in total volume.

Each solution was filtered using a membrane filter with a pore size of 0.22 $\mu$m, and 2 mL of the filtrate was filled into each of 20 barium-free colorless ampoules, followed by melt sealing. Subsequently, the ampoules were subjected to a high-pressure steam sterilization to prepare respective aqueous preparations containing various buffers.

In the samples, pH buffers of glycine, L-valine, L-arginine, L-histidine all reference compounds, acetic acid, malonic acid, maleic acid, succinic acid reference compound, malic acid, tartaric acid, citric acid, sodium glutamate, disodium phthalate, and disodium fumarate reference compound were used in amounts so as to adjust the concentrations to 0, 0.01, 0.05, 0.1, 0.2, and 1.0 wt/vol%.

For each of the samples, the pH before and after the high-pressure steam sterilization was measured, and a buffer capacity (%), an indication of buffer action, was calculated using the following equation and evaluated. The results are shown in Table 2.

```
Buffer capacity (%) = (difference of pH before and after sterilization of sample without buffer - difference of pH before and after sterilization of sample with buffer) / (difference of

pH before and after sterilization of sample without buffer)
```

Table 2

Test results of the buffer capacity (%) calculated from the pH before and after the high-pressure steam sterilization of samples added with various buffers

| Buffer added to sample | Concentration of buffer in sample (wt/vol%) | | | | |
|---|---|---|---|---|---|
| | 0.01 | 0.05 | 0.1 | 0.2 | 1.0 |
| Glycine R.C. | 11.3 | 40.8 | 59.2 | 74.6 | 91.5 |
| L-valine R.C. | -1.5 | 15.4 | 36.9 | 49.2 | 90.8 |
| L-arginine R.C. | 3.2 | 20.6 | 39.7 | 50.8 | 93.7 |
| L-histidine R.C. | 0 | 22.4 | 58.2 | 80.6 | 100.0 |
| Acetic acid | 4.2 | 21.1 | 36.6 | 50.7 | 84.5 |
| Malonic acid | 6.7 | 21.3 | 36.0 | 50.7 | 104.0 |
| Maleic acid | 11.3 | 28.2 | 52.1 | 66.2 | 91.5 |
| Succinic acid R.C. | 12.9 | 30.0 | 44.3 | 65.7 | 87.1 |
| Malic acid | 10.6 | 15.2 | 18.2 | 33.3 | 119.7 |
| Tartaric acid | 10.1 | 30.4 | 36.2 | 49.3 | 95.7 |
| Citric acid | 18.1 | 16.7 | 12.5 | 63.9 | 98.6 |
| Disodium phthalate | 4.3 | 18.8 | 24.6 | 39.1 | 76.8 |
| L-sodium glutamate | 7.4 | 17.6 | 41.2 | 51.5 | 88.2 |

(continued)

| Buffer added to sample | Concentration of buffer in sample (wt/vol%) | | | | |
|---|---|---|---|---|---|
| Disodium fumarate R.C. | 1.4 | 8.2 | 11.0 | 16.4 | - |
| (Note) Each value represents buffer capacity (%). R.C.: reference compound | | | | | |

As shown in Table 2, buffer capacity was dependent on concentration in the range of from 0.01 wt/vol% to 1.0 wt/vol% for each of the buffers except for buffer concentrations of 0.01 wt/vol% of L-valine, 0.01 wt/vol% of L-histidine, and 1.0 wt/vol% of disodium fumarate.

Example 2

An aqueous ferric chloride solution obtained by dissolving 94.6 g of ferric chloride into 390 mL of purified water were fed into a solution of shark chondroitin sulfate obtained by dissolving 97.74 g of shark-cartilage-derived sodium chondroitin sulfate (molecular weight of about 10,000) into 1480 mL of purified water, in a 5-fold amount of the shark-cartilage-derived sodium chondroitin sulfate to the amount of iron (Fe) in weight ratio, with stirring, while adding an amount of an aqueous sodium hydroxide solution necessary to maintain the pH at about 6.5, followed by purified water to adjust to a volume of 5000 mL. Subsequently, the resultant mixture was heated at 110□C for 20 minutes to prepare chondroitin iron sulfate colloid solution with 4 mg/mL of iron concentration.

8.625 g of zinc sulfate was dissolved in 100 mL of purified water to prepare a zinc sulfate solution. Also, 0.624 g of copper sulfate and 0.083 g of potassium iodide were each dissolved in 25 mL of purified water to prepare a copper sulfate solution and a potassium iodide solution.

To 250 mL portions of the chondroitin iron sulfate colloid solution, an appropriate amount of purified water was added, and a pH buffer of malic acid or citric acid corresponding to a concentration of 0.2 wt/vol% was added. After adjusting the pH to about 5.8, the zinc sulfate solution and the copper sulfate solution were added in order, and the pH value was adjusted to 5.8 by adding an aqueous sodium hydroxide solution (1 wt/vol%). Further, the potassium iodide solution was added, and purified water was added to obtain solutions of 1,000 mL in total volume each.

Each solution was filtered using a membrane filter with a pore size of 0.22 $\mu$m, and 2 mL of the filtrate was filled into each of 215 barium-free colorless ampoules, followed by melt sealing. Subsequently, the ampoules were subjected to a high-pressure steam sterilization to prepare aqueous preparations containing respective buffers of malic acid or citric acid.

For the obtained samples, stability evaluation at 70°C storage was conducted. The results are shown in Table 3.

Table 3

| Buffer | Measurement item | | Initial value | 70°C | | |
|---|---|---|---|---|---|---|
| | | | | 10 days | 20 days | 30 days |
| Malic acid | Property | | dark reddish-brown colloid solution, showed Tyndall phenomenon with transmitted light | ← | ← | ← |
| | pH value | | 5.32 | 5.11 | 5.01 | 4.82 |
| | Insoluble contaminant inspection | | clear, no insoluble contaminant | clear, no insoluble contaminant | clear, no insoluble contaminant | clear, no insoluble contaminant |
| | Content (%) | Fe | 98.6 | - | - | 97.7 |
| | | Zn | 99.5 | - | - | 98.5 |
| | | Cu | 101.3 | - | - | 101.3 |

(continued)

| Buffer | Measurement item | | Initial value | 70°C | | |
|---|---|---|---|---|---|---|
| | | | | 10 days | 20 days | 30 days |
| | | I | 99.7 | - | - | 99.0 |
| Citric acid | Property | | dark reddish-brown colloid solution, showed Tyndall phenomenon with transmitted light | ← | ← | ← |
| | pH value | | 5.41 | 5.27 | 5.14 | 4.94 |
| | Insoluble contaminant inspection | | clear, no insoluble contaminant | clear, no insoluble contaminant | clear, no insoluble contaminant | clear, no insoluble contaminant |
| | Content (%) | Fe | 99.1 | - | - | 98.1 |
| | | Zn | 100.0 | - | - | 100.1 |
| | | Cu | 101.6 | - | - | 101.0 |
| | | I | 99.7 | - | - | 97.9 |
| (Note 1) The item of property is based on n = 1 (where n is the number of samples); the items of pH and content show the mean at the time of n = 3; and the item of insoluble contaminant inspection shows the observation results when n = 3. | | | | | | |
| (Note 2) Content was measured by: atomic absorption spectrometry for Fe with ferric chloride, Zn with zinc sulfate, and Cu with copper sulfate; and the HPLC method for I with potassium iodide | | | | | | |

As shown in Table 3, the decline of the pH for each of the samples was mild. On the other hand, for insoluble contaminant inspection, no insoluble contaminant was recognized, and for property and content, almost no change was recognized compared to the initial values.

Each of the samples to which malic acid or citric acid was added as a buffer was stable to extreme temperature as well.

Example 3

An aqueous ferric chloride solution obtained by dissolving 94.6 g of ferric chloride into 390 mL of purified water were fed into a solution of shark chondroitin sulfate obtained by dissolving 97.74 g of shark-cartilage-derived sodium chondroitin sulfate (molecular weight of about 10,000) into 1480 mL of purified water, in a 5-fold amount of the shark-cartilage-derived sodium chondroitin sulfate to the amount of iron (Fe) in weight ratio, with stirring, while adding an amount of an aqueous sodium hydroxide solution necessary to maintain the pH at about 6.5, followed by purified water to adjust to a volume of 5000 mL. Subsequently, the resultant mixture was heated at 110°C for 20 minutes to prepare chondroitin iron sulfate colloid solution with 4 mg/mL of iron concentration.

3.450 g of zinc sulfate was dissolved in 40 mL of purified water to prepare a zinc sulfate solution. In addition, 0.0396 g of manganese chloride, 0.2496 g of copper sulfate, 0.0129 g of selenious acid, and 0.0332 g of potassium iodide were each dissolved in 10 mL of purified water to prepare respective solutions.

To 100 mL of the chondroitin iron sulfate colloid solution, an appropriate amount of purified water was added, and a pH buffer was added. After adjusting the pH to about 5.8, the manganese chloride solution, the zinc sulfate solution, the copper sulfate solution, and the selenious acid solution were added in order, and the pH value was adjusted to 5.8 by adding an aqueous sodium hydroxide solution (1 wt/vol%). Further, the potassium iodide solution was added, and purified water was added to obtain a solution of 400 mL in total volume.

The solution was filtered using a membrane filter with a pore size of 0.22 $\mu$m, and 2 mL of the filtrate was filled into each of 10 barium-free colorless ampoules, followed by melt sealing. Subsequently, the ampoules were subjected to a high-pressure steam sterilization to prepare aqueous preparations.

As samples, three kinds of aqueous preparations each containing six elements (iron, manganese, zinc, copper, selenium and iodine) with 0.1 wt/vol% of glycine, and 0.2 wt/vol% of citric acid and sodium glutamate were prepared.

For these samples, a mixing test was conducted after mixing 2 mL of the solution to the following high-calorie intravenous nutrition infusions.

For the mixing test, observation was performed with respect to the properties, the pH, and the insoluble contaminant at room temperature just after mixing and 24 hours after mixing. The results of this test are shown in Table 4. The commercially available infusions used were "AMINOTRIPA No.2" (trade name, Otsuka Pharmaceutical Co., Ltd.) and "PNTWIN-3" (trade name, Ajinomoto Pharma Co., Ltd.).

Table 4

| Infusion | Aqueous preparation | Test item | Before mixing | Just after mixing | 24 hours after mixing |
|---|---|---|---|---|---|
| AMINO-TRIPA No. 2 | Containing glycine (reference) | Property | Clear and colorless | Clear yellowish brown | Clear yellowish brown |
| | | pH value | 5.58 | 5.58 | 5.51 |
| | | Insoluble contaminant | None | None | None |
| | Containing citric acid | Property | Clear and colorless | Clear yellowish brown | Clear yellowish brown |
| | | pH value | 5.59 | 5.59 | 5.51 |
| | | Insoluble contaminant | None | None | None |
| | Containing L-sodium glutamate | Property | Clear and colorless | Clear yellowish brown | Clear yellowish brown |
| | | pH value | 5.60 | 5.59 | 5.52 |
| | | Insoluble contaminant | None | None | None |
| PNTWIN-3 | Containing glycine (reference) | Property | Clear and colorless | Clear yellowish brown | Clear yellowish brown |
| | | pH value | 5.16 | 5.18 | 5.14 |
| | | Insoluble contaminant | None | None | None |
| | Containing citric acid | Property | Clear and colorless | Clear yellowish brown | Clear yellowish brown |
| | | pH value | 5.18 | 5.16 | 5.15 |
| | | Insoluble contaminant | None | None | None |
| | Containing L-sodium glutamate | Property | Clear and colorless | Clear yellowish brown | Clear yellowish brown |
| | | pH value | 5.18 | 5.18 | 5.15 |
| | | Insoluble contaminant | None | None | None |

As shown in Table 4, each of the aqueous preparations of the present invention (Example 3) did not show any change in its properties up to 24 hours after mixing in the commercially available infusions, and precipitates such as insoluble contaminants were not observed. In addition, almost no change in pH was recognized.

Example 4

Similar to the Examples above, an aqueous ferric chloride solution obtained by dissolving 94.6 g of ferric chloride into 390 mL of purified water were fed into a solution of shark chondroitin sulfate obtained by dissolving 97.74 g of shark-cartilage-derived sodium chondroitin sulfate (molecular weight of about 10,000) into 1480 mL of purified water, in a 5-fold amount of the shark-cartilage-derived sodium chondroitin sulfate to the amount of iron (Fe) in weight ratio, with stirring, while adding an amount of an aqueous sodium hydroxide solution necessary to maintain the pH at about 6.5, followed by purified water to adjust to a volume of 5000 mL. Subsequently, the resultant mixture was heated at 110°C for 20 minutes to prepare chondroitin iron sulfate colloid solution with 4 mg/mL of iron concentration. Subsequently, heat treatment was carried out to prepare a chondroitin iron sulfate colloid solution with 4 mg/mL of iron concentration.

172.50 g of zinc sulfate was dissolved in 2,000 mL of purified water to prepare a zinc sulfate solution. In addition, 12.48 g of copper sulfate and 1.66 g of potassium iodide were each dissolved in 500 mL of purified water to prepare respective solutions.

To 5,000 mL of the chondroitin iron sulfate colloid solution, an appropriate amount of purified water was added. After adjusting the pH to about 6, the zinc sulfate solution, and the copper sulfate solution were added in order, and the pH was adjusted to 5.8 by adding an aqueous sodium hydroxide solution (1 wt/vol%). Further, the potassium iodide solution was added and purified water was added to obtain a solution of 20,000 mL in total volume.

The solution was filtered using a membrane filter with a pore size of 0.22 $\mu$m, and 2 mL of the filtrate was filled into each of 445 barium-free colorless ampoules, followed by melt sealing. Subsequently, the ampoules were subjected to a high-pressure steam sterilization to prepare aqueous preparations.

The prepared preparations were confirmed to be, by conducting stability evaluation for 6 months at 40°C, stable with respect to the property, the pH, insoluble contaminant inspection, insoluble fine particle test, and content.

Effect of the Invention

The aqueous preparation of the present invention can be safely applied in clinical use without worry of BSE infection. It is pharmaceutically stable, and is stable in an infusion such as a high-calorie intravenous nutrition infusion.

**Claims**

1.  A stabilized aqueous preparation comprising a shark-derived chondroitin iron sulfate colloid, a trace element and a pH buffer, wherein the shark-derived chondroitin iron sulfate colloid is produced from a shark-derived sodium chondroitin sulfate which has an average molecular weight of from 10,000 to 25,000; a limiting viscosity of from 0.27 to 0.65 dl/g; and a sulfur content of from 6.4 to 7.0 wt/wt% and wherein the pH buffer is one or more compounds selected from the group consisting of acetic acid, malonic acid, maleic acid, malic acid, tartaric acid, citric acid, L-sodium glutamate and disodium phthalate.

2.  An aqueous preparation according to Claim 1, wherein the pH buffer is malic acid, citric acid or L-sodium glutamate.

3.  An aqueous preparation according to Claim 2, wherein the pH buffer is one or both of malic acid and citric acid.

4.  An aqueous preparation according to Claim 1, wherein the trace element is one or more elements selected from the group consisting of selenium, zinc, copper, iodine, and manganese.

5.  An aqueous preparation according to Claim 4, wherein the trace element consists of zinc, copper, and iodine.

6.  An aqueous preparation according to Claim 4, wherein the trace element consists of zinc, copper, iodine, and manganese.

7.  An aqueous preparation according to Claim 4, wherein the trace element consists of selenium, zinc, copper, iodine, and manganese.

8.  An aqueous preparation according to Claim 1, wherein a weight ratio of iron : the shark-derived sodium chondroitin iron sulfate colloid is from 1:4 to 1:6.

9. An aqueous preparation according to Claim 8, wherein the weight ratio of the iron : the shark-derived chondroitin iron sulfate colloid is 1:5.

10. An aqueous preparation according to Claim 1, wherein the concentration of the pH buffer is from 0.01 to 1.0 wt/vol%.

11. An aqueous preparation according to Claim 1, wherein the preparation contains the shark-derived chondroitin iron sulfate colloid produced by adding an aqueous ferric salt solution and an aqueous alkali metal hydroxide solution to an aqueous shark-derived chondroitin sulfate solution so that a pH of the resulting mixture is adjusted to a pH within a range of from 5.5 to 7.5.

12. A method for manufacturing an aqueous preparation as defined in Claim 1, comprising: adding an aqueous ferric salt solution and an aqueous sodium hydroxide solution to an aqueous shark-derived chondroitin sulfate solution so that a pH of the resulting mixture is adjusted to a pH within a range of from 5.5 to 7.5; heating the resulting mixture; and adding a pH buffer and a trace element.

13. A method for manufacturing an aqueous preparation containing a shark-derived chondroitin iron sulfate colloid according to Claim 12, wherein the shark-derived chondroitin sulfate is shark-derived sodium chondroitin sulfate, and the alkali metal hydroxide is sodium hydroxide.

**Patentansprüche**

1. Stabilisierte wässrige Zubereitung, umfassend von Hai abgeleitetes Chondroitineisensulfat-Kolloid, ein Spurenelement und einen pH-Puffer, wobei das von Hai abgeleitete Chondroitineisensulfat-Kolloid aus einem von Hai abgeleiteten Natriumchondroitinsulfat produziert wird, das ein durchschnittliches Molekulargewicht von 10.000 bis 25.000, eine Grenzviskosität von 0,27 bis 0,65 dl/g und einen Schwefelgehalt von 6,4 bis 7,0 Gewichts-% hat, und wobei der pH-Puffer eine Verbindung oder mehrere Verbindungen ist, die aus der Gruppe bestehend aus Essigsäure, Malonsäure, Maleinsäure, Äpfelsäure, Weinsäure, Citronensäure, L-Natriumglutamat und Dinatriumphthalat, ausgewählt ist/sind.

2. Wässrige Zubereitung nach Anspruch 1, wobei der pH-Puffer Äpfelsäure, Citronensäure oder L-Natriumglutamat ist.

3. Wässrige Zubereitung nach Anspruch 2, wobei der pH-Puffer eine oder beide von Äpfelsäure und Citronensäure ist.

4. Wässrige Zubereitung nach Anspruch 1, wobei das Spurenelement ein Element oder mehrere Elemente, ausgewählt aus der Gruppe, bestehend aus Selen, Zink, Kupfer, Iod und Mangan, ist.

5. Wässrige Zubereitung nach Anspruch 4, wobei das Spurenelement aus Zink, Kupfer und Iod besteht.

6. Wässrige Zubereitung nach Anspruch 4, wobei das Spurenelement aus Zink, Kupfer, Iod und Mangan besteht.

7. Wässrige Zubereitung nach Anspruch 4, wobei das Spurenelement aus Selen, Zink, Kupfer, Iod und Mangan besteht.

8. Wässrige Zubereitung nach Anspruch 1, wobei das Gewichtsverhältnis von Eisen : von Hai abgeleitetes Natriumchondroitineisensulfat-Kolloid von 1:4 bis 1:6 ist.

9. Wässrige Zubereitung nach Anspruch 8, wobei das Gewichtsverhältnis Eisen : von Hai abgeleitetes Chondroitineisensulfat-Kolloid 1:5 ist.

10. Wässrige Zubereitung nach Anspruch 1, wobei die Konzentration des pH-Puffers 0,01-1,0 G/V% ist.

11. Wässrige Zubereitung nach Anspruch 1, wobei die Zubereitung das von Hai abgeleitete Chondroitineisensulfat-Kolloid enthält, das hergestellt wurde, indem eine wässrige Eisen(III)-salz-Lösung und eine wässrige Alkalimetallhydroxidlösung zu einer wässrigen von Hai abgeleitetem Chondroitinsulfat-Lösung so zugesetzt wird, dass der pH des resultierenden Gemisches auf einen pH innerhalb des Bereichs von 5,5 bis 7,5 eingestellt wird.

**12.** Verfahren zur Herstellung einer wässrigen Zubereitung, wie sie in Anspruch 1 definiert ist, umfassend: Zugeben einer wässrigen Eisen(III)-salz-Lösung und einer wässrigen Natriumhydroxidlösung zu einer wässrigen Lösung von Hai abgeleitetem Chondroitinsulfat derart, dass der pH des resultierenden Gemisches auf einen pH innerhalb des Bereichs von 5,5 bis 7,5 eingestellt wird; Erwärmen des resultierenden Gemisches und Zugeben eines pH-Puffers und eines Spurenelements.

**13.** Verfahren zur Herstellung einer wässrigen Zubereitung, die ein von Hai abgeleitetes Chondroitineisensulfat-Kolloid nach Anspruch 12 enthält, wobei das von Hai abgeleitete Chondroitinsulfat Natriumchondroitinsulfat ist und das Alkalimetallhydroxid Natriumhydroxid ist.

**Revendications**

**1.** Préparation aqueuse stabilisée comprenant un colloïde de chondroïtine sulfate de fer extrait de requin, un oligoélément et un tampon de pH, dans laquelle le colloïde de chondroïtine sulfate de fer extrait de requin est produit à partir de chondroïtine sulfate de sodium extrait de requin qui a un poids moléculaire moyen de 10.000 à 25.000, une viscosité limite de 0,27 à 0,65 dl/g ; et une teneur en soufre de 6,4 à 7,0% en poids/poids et dans laquelle le tampon de pH est un ou plusieurs composés choisis dans le groupe consistant en acide acétique, acide malonique, acide maléique, acide malique, acide tartrique, acide citrique, L-glutamate de sodium et phtalate disodique.

**2.** Préparation aqueuse selon la revendication 1, dans laquelle le tampon de pH est l'acide malique, l'acide citrique ou le L-glutamate de sodium.

**3.** Préparation aqueuse selon la revendication 2, dans laquelle le tampon de pH est l'un ou l'autre de l'acide malique et de l'acide citrique ou les deux.

**4.** Préparation aqueuse selon la revendication 1, dans laquelle l'oligoélément est un ou plusieurs éléments choisis dans le groupe consistant en sélénium, zinc, cuivre, iode et manganèse.

**5.** Préparation aqueuse selon la revendication 4, dans laquelle l'oligoélément consiste en zinc, cuivre et iode.

**6.** Préparation aqueuse selon la revendication 4, dans laquelle l'oligoélément consiste en zinc, cuivre, iode et manganèse.

**7.** Préparation aqueuse selon la revendication 4, dans laquelle l'oligoélément consiste en sélénium, zinc, cuivre, iode et manganèse.

**8.** Préparation aqueuse selon la revendication 1, dans laquelle le rapport en poids du fer au colloïde de chondroïtine sulfate de fer de sodium extrait de requin est de 1 :4 à 1 :6.

**9.** Préparation aqueuse selon la revendication 8, dans laquelle le rapport en poids du fer au colloïde de chondroïtine sulfate de fer extrait de requin est de 1 :5.

**10.** Préparation aqueuse selon la revendication 1, dans laquelle la concentration en tampon de pH est de 0,01 à 1,0% en poids/volume.

**11.** Préparation aqueuse selon la revendication 1, dans laquelle la préparation contient le colloïde de chondroïtine sulfate de fer extrait de requin produit par addition aqueuse d'une solution de sel ferrique et d'une solution aqueuse d'hydroxyde de métal alcalin à une solution aqueuse de chondroïtine sulfate extrait de requin de telle sorte que le pH du mélange résultant est ajusté à un pH compris dans une gamme de 5,5 à 7,5.

**12.** Procédé de fabrication d'une préparation aqueuse selon la revendication 1, comprenant : l'addition d'une solution aqueuse de sel ferrique et d'une solution aqueuse d'hydroxyde de sodium à une solution aqueuse de chondroïtine sulfate extrait de requin de telle sorte que le pH du mélange résultant est ajusté à un pH compris dans une gamme de 5,5 à 7,5 ; le chauffage du mélange résultant ; et l'addition d'un tampon de pH et d'un oligoélément.

**13.** Procédé de fabrication d'une préparation aqueuse contenant un colloïde de chondroïtine sulfate de fer extrait de requin selon la revendication 12, dans lequel le chondroïtine sulfate extrait de requin est un chondroïtine sulfate de sodium extrait de requin, et l'hydroxyde de métal alcalin est l'hydroxyde de sodium.